# EUROPEAN PATENT SPECIFICATION

(11) **EP 3 278 767 B1**
(45) Date of publication and mention of the grant of the patent: **28.02.2024**
(21) Application number: 17275120.8
(22) Date of filing: 03.08.2017
(51) Int. Cl.: A61F 2/07, A61F 2/06

(54) **AN ENDOGRAFT FOR TREATING BRANCHED VESSELS**
ENDOGRAFT ZUR BEHANDLUNG VON VERZWEIGTEN BLUTGEFÄSSEN
ENDOGREFFE SERVANT À TRAITER DES VAISSEAUX RAMIFIÉS

(30) Priority: 04.08.2016 US 201615228438; 04.08.2016 AU 2016210717
(43) Date of publication of application: 07.02.2018
(73) Proprietor: Cook Medical Technologies LLC, Bloomington, IN 47404 (US)
(72) Inventor: KING, Chantelle, Woodridge, Queensland 4114 (AU); ROEDER, Blayne A., Bloomington, IN 47401 (US)
(74) Representative: Williams Powell

(56) References cited:
- EP-A1- 2 547 287
- EP-A1- 3 470 017
- EP-B1- 2 119 416
- EP-B1- 2 547 287
- WO-A1-2006/065644
- WO-A2-03/082153
- US-A1- 2011 276 062
- US-B1- 6 645 242

## Description

### TECHNICAL FIELD

The present disclosure relates generally to medical devices. Embodiments relate to endografts. Embodiments relate to endografts deployable into the vascular system of humans or animals.

### BACKGROUND

US 6,645,242 discloses a bifurcated side-access intravascular stent graft.

Endografts and delivery device assemblies for endografts are used in aortic intervention. They are used by vascular surgeons to treat aneurysms and to repair regions of the aorta, including the aortic arch, the thoracic aorta, the abdominal aorta and the aortic bifurcation.

Multiple stent grafts may be implanted to provide a system of interconnected stent grafts. Interconnected stent grafts can be made of fenestrated stent grafts and smaller side branch grafts.

Endografts for treating branched vessels, typically stent grafts, are intricate and take considerable time to produce.

Throughout this specification, the term "distal" with respect to a portion of the aorta, a deployment device or an endograft means the end of the aorta, deployment device or endograft further away in the direction of blood flow from the heart and the term "proximal" means the portion of the aorta, deployment device or end of the endograft nearer to the heart in the direction of blood flow. When applied to other vessels, similar terms such as caudal and cranial should be understood.

### SUMMARY

Aspects of the present invention seek to provide an improved endograft, for example for treating branched vessels. Aspects of the present invention seek to provide an improved method.

According to an aspect of the invention, there is provided an endograft as in claim 1.

According to an aspect of the invention, there is provided a method as in claim 13.

According to an aspect of the invention, there is provided an endograft comprising a proximal end and a distal end, the endograft comprising a tube of biocompatible material, the tube comprising:
a main lumen extending longitudinally from a proximal end to a distal end, the main lumen including a proximal portion, an intermediate portion and a distal portion;
a branch lumen within the intermediate portion of the main lumen; and
a seam, the seam extending longitudinally beside both the branch lumen and the distal portion of the main lumen,
wherein the tube comprises a lateral cross-section defining a tubular wall, wherein a portion of the tubular wall is pinched together by a seam to form an internal branch portion.

According to an aspect of the present disclosure, there is provided an endograft comprising a proximal end and a distal end, the endograft comprising a tube of biocompatible material, the tube comprising: a main lumen extending longitudinally from the proximal end to the distal end, the main lumen including a proximal portion, an intermediate portion and a distal portion; a branch lumen within the intermediate portion of the main lumen; and a seam, the seam extending longitudinally beside both the branch lumen and the distal portion of the main lumen, wherein the tube comprises a lateral cross-section defining a tubular wall pinched together by the seam to form a branch portion.

According to an aspect of the present invention, there is provided a method of producing a endograft comprising the steps of: providing a tube of biocompatible material, the tube having a tubular wall; flattening the tube so that the tubular wall forms two substantially planar wall portions; partitioning the flattened tube by creating a longitudinal seam between the two wall portions; cutting a piece out of the tube from a position adjacent to the longitudinal seam so as to create a branch mouth into the tube; attaching a super-elastic ring to the branch mouth; and everting the tube.

### BRIEF DESCRIPTION OF DRAWINGS

Embodiments of the present disclosure are described below, by way of example only, with reference to the accompanying drawings wherein:
Figure 1 is a plan view of a flattened tube of biocompatible material with a 12 o'clock line down its centre;
Figure 2 is a similar view to that of Figure 1 but shows the 12 o'clock line on the right hand lateral edge of the tube;
Figure 3A is an end view of the tube of Figure 2 after a longitudinally extending portion from the tubular wall has been cut out;
Figure 3B is similar view to Figure 3A but shows the tube after edges formed from a cut have been sealed;
Figure 4A is an isometric view showing the tube of Figure 3;
Figures 4B, 4C and 4D are proximal lateral, intermediate lateral and distal lateral cross-sections through the tube shown in Figure 4A through section lines 4B-4B, 4C-4C and 4D-4D respectively;
Figure 5A shows an endograft according to the disclosure is an isometric view;
Figures 5B, 5C and 5D show lateral cross-sections of the endograft of Figure 5A through section lines 5B-5B, 5C-5C and 5D-5D respectively;
Figure 6 is a longitudinal cross-sectional view of the endograft of Figure 5A;
Figure 7 is an isometric view looking into the endograft of Figures 5A and 6 from its proximal end;
Figure 8 is an isometric view of a space frame;
Figure 9 is an isometric view showing the space frame of Figure 8 attached to a portion of the endograft of Figure 4A;
Figure 10A is a diagrammatic view of the endograft of Figures 5A, 6 and 7 within the anatomy of a patient;
Figure 10B is a similar view to that of Figure 10A but also shows a side branch endograft; and
Figure 11 is an isometric view of another endograft according to the disclosure.

### DESCRIPTION OF EMBODIMENTS

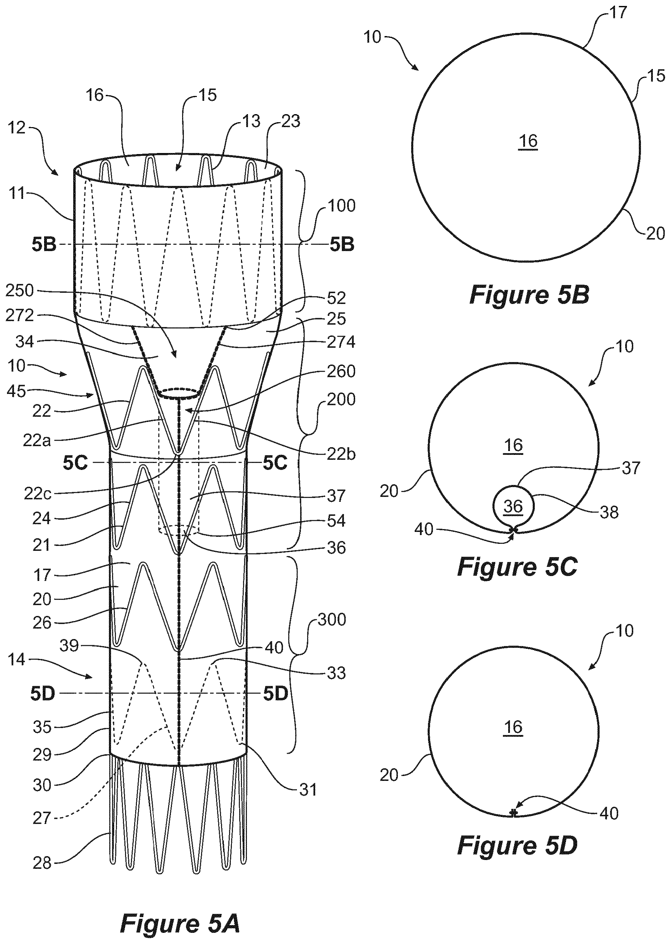

One example of an endograft 10 according to the present disclosure is shown in Figure 5A. Figures 5B, 5C and 5D show lateral cross-sections of the endograft of Figure 5A through section lines 5B-5B, 5C-5C and 5D-5D respectively.

Referring to Figure 5A, it can be seen that the endograft 10 consists generally of in the form of a tubular stent graft having a proximal end portion 12 and a distal end portion 14, a tubular body 15, and a lumen 16 extending therethrough. The endograft 10 may be configured to receive a fluid flow in a proximal to distal direction.

The endograft 10 may be any suitable length. In one example, the endograft 10 is a suitable length corresponding to the length of a lesion site where the endograft 10 is to be positioned.

The endograft 10 may be in a compressed or collapsed configuration (not shown) or a radially expanded configuration (shown in Figure 5A). In the expanded configuration the endograft 10 may apply a radially outward force upon at least a portion of a vessel, duct, or lumen e.g., to maintain patency within a passageway.

The endograft 10 may be any suitable selected diameter and may be constructed of any biocompatible graft material 20 which is suitable for facilitating repair of an injured or diseased body vessel. The graft material 20 may be synthetic and/or naturally-derived material. Synthetic biocompatible polymers may include but are not limited to polyethylene terephthalate, polyurethane, nylon, polyester, high molecular weight polyethylene (such as Thoralon), polytetrafluoroethylene, or combinations thereof. The graft material 20 can be porous or non-porous and also may be impregnated or coated with one or more therapeutic substances. In one example, the graft material may be constructed of the commercially available material referred to as Dacron. In another example the graft material may consist of small-intestine submucosa ("SIS") material, which may be obtained from porcine intestine. The graft material should have sufficient flexibility to allow for navigation of the vasculature and delivery to a targeted area in the body. Preferably, the graft material 20 is a low profile material or an ultralow profile material.

The endograft 10 may be supported by one or more stents 21 along its length. One or more stents 21 may be located on an interior surface 23, exterior surface 25, or both, of the tubular body 15 of the endograft 10. The number of stents and their placement (internal or external) can be varied to suit the particular application or patient anatomy. Alternatively, the endograft 10 may be unsupported along its length such that there are no body stents located on the graft material between the proximal end portion 12 and distal end portion 14 of the endograft 10.

In one example, stent 21 may be a Z-stent. For example, stent 21 may have a distal end 30 with a series of distal apices 31 and a proximal end 39 with a series of proximal apices 33. Stent 21 may also have one or more elongate struts 35 connecting the distal apices 31 to the proximal apices 33.

Suitable stents 21 for use in connection with the endograft 10 described herein may be self-expanding or mechanically-expandable stents or both, and may be deployed according to conventional methodology. A self-expanding stent may be manufactured from a shape-memory alloy, such as nickel titanium alloy (Nitinol). If the stent comprises a self-expanding material such as Nitinol, the stent may be heat-set into the desired expanded state whereby the stent can assume a relaxed radially expanded configuration. The stent may be made from other metals and alloys that allow the stent to return to its original expanded configuration upon deployment, such as, for example, stainless steel, cobalt-chrome alloys, amorphous metals, and/or non-metallic materials as would be recognized by one of skill in the art. Additionally or alternatively, the endograft 10 may be mechanically expanded, such as through the use of an expandable balloon placed within the lumen 16 of the endograft 10 and then radially outwardly expanded.

In the example shown in Figure 5A, a plurality of zig zag stents 21 include a proximal end internal stent 13 and a distal end internal stent 27. Between stents 13 and 27 they may be three external stents 22, 24, 26.

The endograft 10 may be anchored to an interior wall of a body vessel, duct, or lumen proximally and/or distally to a lesion site. The endograft 10 of Figure 5A has a proximal end sealing region 11 and a distal end sealing region 29. For example, the distal sealing region 29 of the endograft 10 of Figure 5A may include an uncovered distal stent 28. This distal stent 28 may include barbs (not shown) to anchor the endograft 10 in place.

The tubular body 15 of the endograft 10 may have a tubular wall 17 extending from the proximal end portion 12 to the distal end portion 14 of the endograft 10. The diameter of the tubular body 15 may change along the length of the endograft 10. In one example, the diameter at the proximal end portion 12 of the tubular body 15 is greater than the diameter at the distal end portion 14 of the tubular body 15. A tapered transition portion 45 may connect the proximal end portion 12 and the distal end portion 14.

The tubular body 15 may have one or more lumens extending therethrough. In one example, the tubular body 15 comprises the main lumen 16 extending longitudinally from the proximal end portion 12 to the distal end portion 14 of the endograft 10.

The endograft 10 includes an internal branch portion 37. In one example, the internal branch portion 37 extends from a recess or opening 250 in the tubular wall 17 into the main lumen 16 of the tubular body 15. The branch portion 37 may have a proximal end 52, and distal end 54 and a lumen 36 extending therethrough. In one example, the proximal end 52 has a larger diameter than the distal end 54 of the branch portion 37. The branch portion 37 may be constructed from the same biocompatible material as the graft material 20.

Figures 5B, 5C, and 5D show the endograft 10 at the proximal lateral 100, intermediate lateral 200 and distal lateral 300 cross-sections, respectively. These lateral cross-sections can be identified as section lines 5B-5B, 5C-5C and 5D-5D marked on Figure 5A respectively. As shown in Figure 5B, the tubular wall has the main lumen 16 extending therethrough at the proximal lateral portion 100 of the endograft 10. In the intermediate lateral 200 cross section (shown in Figure 5C), the main lumen 16 and the lumen 36 of the internal branch portion 37 extend through the tubular body 15. Figure 5C also shows a seam 40 in the graft material 20 which will be described in greater detail below. In the distal lateral 300 cross-section (Figure 5D), the main lumen 16 extends through the endograft 10.

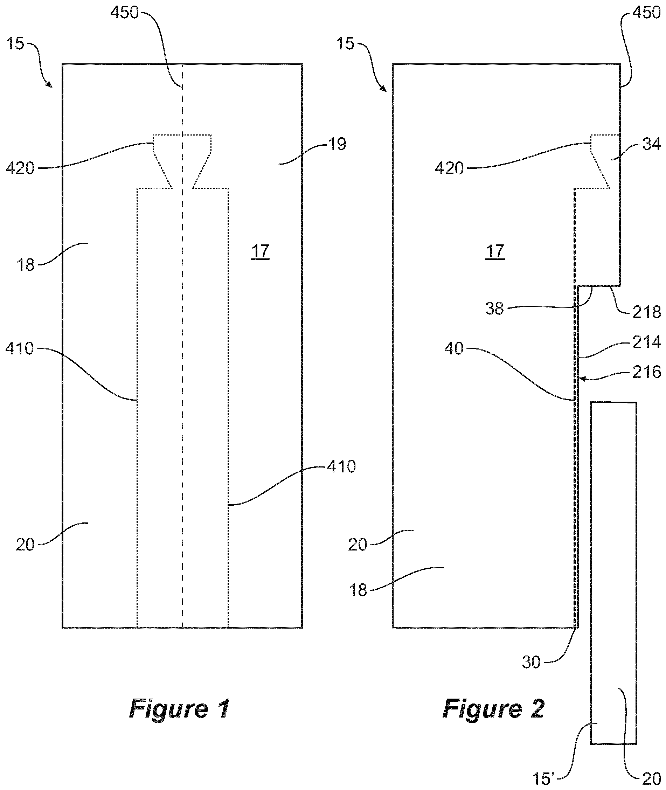

In order to better understand the structure and construction of the endograft shown in Figures 5A to 5D, one method of constructing the endograft 10 shown in Figure 5A will now be described with reference to Figures 1-4. In particular, one novel feature of the disclosed endograft 10 is that the internal branch portion 37 is constructed using the excess of the graft material 20 used to make the tubular body 15, thus eliminating or reducing the need to sew a separate branch to the graft material.

Figure 1 is a plan view of a flattened tube of biocompatible material with a 12 o'clock line down its centre. Figure 2 is a similar view to that of Figure 1 but shows the 12 o'clock line on the right hand lateral edge of the tube.

Referring to Figure 1, a tube of biocompatible material that may form the tubular body 15 is shown. In particular, tubular wall 17 of the endograft 10 is shown in a flattened condition so as to form two parallel spaced-apart folds that define tubular wall portion 18 and tubular wall portion 19. Referring to Figure 1, a 12 o'clock line 450 is marked on the graft material 20. Further lines 410 and 420 are marked on the graft material 20. Lines 410 and 420 may represent a portion of the outline of the internal branch portion 37. The line 420 indicates the position that will become the recess or opening 250 and the exit 34 from the branch lumen 36.

Figure 2 shows the graft material 20 folded at the 6 and 9 o'clock position aligning the lines 410 with each other.

As shown in Figure 2, the flattened graft material may be partitioned by sewing the seam 40 along the internal branch lines 410. In other words, the seam 40 may attach two portions of the graft material 20 together along line 410. The seam 40 may be sewn using any suitable method and may be sewn using any suitable biocompatible material.

After the seam 40 is sewn, an excess portion 15' of graft material 20 may be cut out and discarded. In one example, a longitudinal cut may occur near the seam 40 at longitudinal edges 214 and 216. Two adjacent longitudinal edges 214 and 216 may join each other along the seam 40. A latitudinal cut may occur at lateral edge 218.

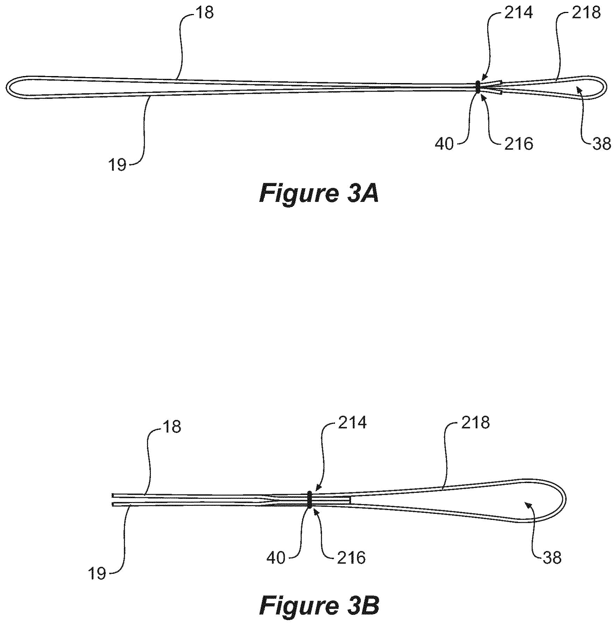

The graft material 20 may be sealed my any means along one or more cut lines. In one example (shown in Fig. 3b), the graft material 20 is heat sealed along longitudinal edges 214 and 216 as described below.

The seam 40 may extend from the distal end portion 30 of the endograft 10 to a point proximal to the lateral edge 218. The branch portion 37 may be formed in the tubular wall 17 area between the seam 40 and the 12 o'clock line 450. The branch portion 37 may have a mouth 38 and an exit 34 that connects the branch portion 37 to the tubular body 15.

Figures 3A and 3B show end views of the flattened tube of Figure 2 after the seam 40 has been sewn and a portion 15' of the graft material 20 has been cut out and discarded. In particular, Figure 3A is an end view of the tube of Figure 2 after a longitudinally extending portion from the tubular wall has been cut out. Figure 3B is a similar view to Figure 3A but shows the tube after edges 214 and 218 formed from a cut have been heat sealed.

As shown in Figures 3A and 3B, the tubular wall portions 18 and 19 are sewn together to form the seam 40. Adjacent longitudinal edges 214 and 216 join each other at the lateral edge 218. All of the edges 214, 216 and 218 are formed when the excess graft material 15' is cut and discarded. This leaves the lateral edge 218 and the mouth 38 of the internal branch portion 37.

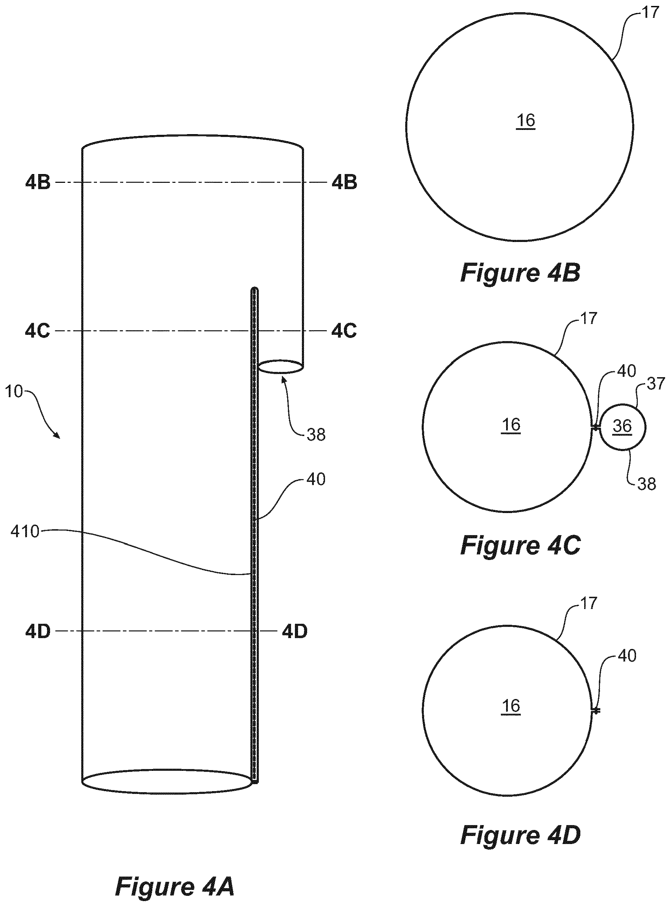

Figure 4A is an isometric view showing the tube of Figure 3B. As shown in Figures 4A, the graft material 20 is no longer in a flattened configuration, but rather in a tubular configuration. The seam 40 occurs along the graft material 20 along line 410 and the excess graft material has been cut away. The mouth 38 to the internal branch portion 37 is illustrated in Figure 4A.

Figures 4B, 4C and 4D are proximal lateral, intermediate lateral and distal lateral cross-sections through the tube shown in Figure 4A through section lines 4B-4B, 4C-4C and 4D-4D respectively. As shown in Figure 4B, the graft material forms the tubular wall 17 with main lumen 16. At the intermediate lateral cross-section, Figure 4C illustrates addition of the branch portion 37 having lumen 36. Figure 4C also shows the seam 40 in the tubular wall 17. At the distal lateral cross section 4D-4D shown in Figure 4D, the tubular wall 17 and the seam 40 are illustrated.

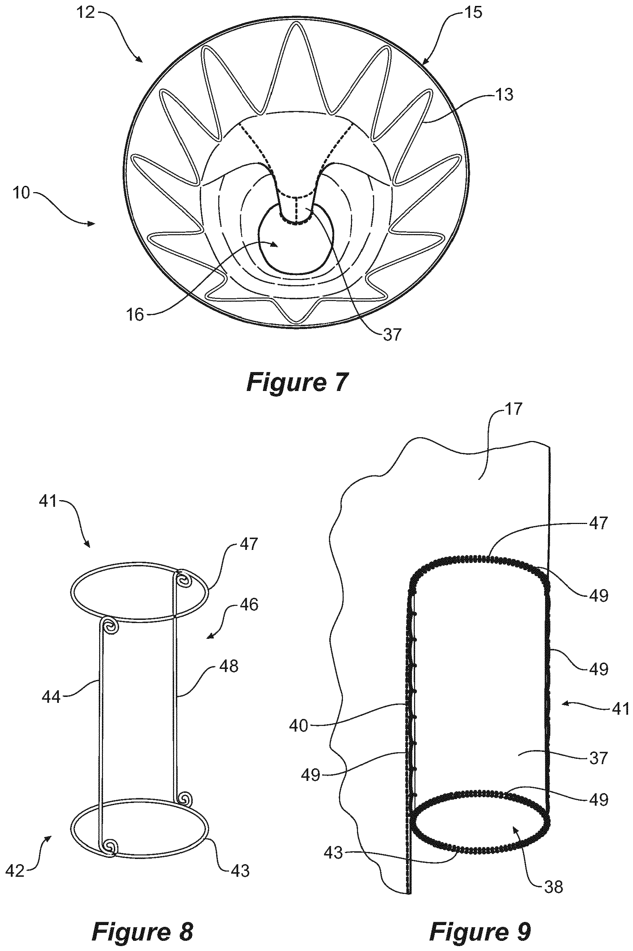

It may be desirable to provide additional support to the internal branch portion 37. In one example, a super-elastic ring of nitinol wire is attached to support the branch portion 37. For example, space frame 41 may provide additional support to the internal branch portion 37. Figure 8 is an isometric view of a space frame. Figure 9 is an isometric view showing the space frame of Figure 8 attached to the internal branch portion 37 shown in Figure 4A.

As shown in Figures 8 and 9, it can be seen that the branch portion 37 may have a space frame 41. The space frame 41 may have an entrance reinforcing ring 43 and an exit reinforcing ring 47. The entrance and exit reinforcing rings 43,47 may be spaced apart axially by one or more struts 44 and 48. In one example, the space frame 41 comprises an assembly of two individual ring and strut components 42, 46. Each ring and strut component 42, 46 may comprise a circular ring portion defining a plane of the circular ring portion and a strut extending at right angles to the plane of the circular ring portion from a periphery of the circular ring portion. One of the two circular rings may form the entrance reinforcing ring 43 and the other of the two circular rings may form the exit reinforcing ring 47. For instance, ring and strut component 42 may comprise the entrance reinforcing ring 43 and the strut 44. Ring and strut component 46 may comprise the exit reinforcing ring 47 and strut 48.

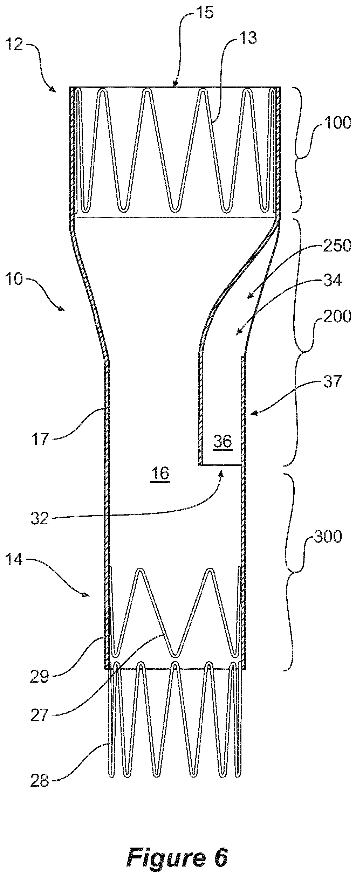

As shown in Figure 9, the space frame 41 may be attached to the branch portion 37 in any suitable way. In one example, space frame 41 is sewn into the branch portion 37 with stitching 49. The mouth 38 of the branch portion 37 may be biased towards an open condition by the entrance reinforcing ring 43. Similarly, the exit reinforcing ring 47 may bias the exit 34 (shown Figure 6) into an open condition. This open condition is also apparent in the isometric view of Figure 7 looking down into the endograft 10 from its proximal end 12.

The space frame 41 may be constructed with any biocompatible material. In one example, the space frame 41 comprises super-elastic wire, such as Nitinol. Other suitable materials may be used.

The tubular body 15 graft material 20 shown in Figures 1-4 and 8-9 may be everted or turned inside out so that the branch portion 37 sits internally in the tubular body 15, as shown in the endograft 10 shown in Figures 5 -7 and 10-11. Figures 5A-D show one example of an endograft 10 after it has been constructed according to the disclosure above.

Returning to Figures 5A-D, once the tubular body 15 graft material 20 is turned inside out, stents 13, 22, 24, 26, 28, and 29 may be attached to the tubular wall 17 by any known method including using adhesives and stitching. Once the branch portion 37 is located internally in the tubular body, the shape of the branch portion 37 may further be changed as desired. For example, the branch portion 37 may be further cut and heat sealed to any desirable shape. In one example, excess tubular wall material may be cut out and the resultant slits may be sewn to form seams 272 and 274. In other words, the excess material may be gathered and trimmed to create a V-shape (as shown in Figure 5A).

As shown in Figure 5A, the recess or opening 250 in the tubular wall 17 may open into the exit 34 of the branch portion 37. The recess or opening 250 may be at least partially disposed within a V-shaped region 260 formed between two adjacent struts 22a, 22b and a bend 22c of zig zag stent 22, that stent 22 being one of the plurality of zig zag stents 21 as is shown most clearly in Figure 5A. In one example, the tubular wall 17 of the main lumen may have at least one seam 40 adjacent to the V-shaped region 260 formed between two adjacent struts and a bend of one of the plurality of zig zag stents 21.

As shown in Figure 5A, the tubular body 15 of the endograft 10 and in embodiments also the main lumen may include a proximal portion 100, an intermediate portion 200 and a distal portion 300. The tubular body 15 of the endograft 10 may have the seam 40 extending longitudinally along a portion of the tubular wall 17. The seam 40 may extend longitudinally beside both the branch lumen and the distal portion of the main lumen.

Figures 5B, 5C and 5D show lateral cross-sections of the endograft of Figure 5A through section lines 5B-5B, 5C-5C and 5D-5D respectively. In the proximal portion (shown in Figure 5B), the tubular wall 17 has the main lumen 16. In the intermediate portion 200 (shown in Figure 5C) the internal branch portion 37 and the branch lumen 38 may be within the main lumen 16. Referring again to Figures 5A, 5B, 5C, 5D and 6, it can be seen that the seam 40 may be created where the tubular wall 17 is pinched together to form the branch portion 37. In the distal portion (shown in Figure 5D), the distal end 14 of the endograft can be seen as well as the seam 40 formed where the tubular wall 17 is stitched after the excess graft material has been cut out and discarded.

Figure 6 is a longitudinal cross-sectional view of the endograft of Figure 5A. Figure 7 is an isometric view looking into the endograft of Figures 5A and 6 from its proximal end.

Referring to the longitudinal cross-sectional view of Figure 6, the internal branch portion 37 can be seen internal to the tubular body 15. With this cross-sectional view, the external stents 22,24,26 and the wires of the space frame 41 are not shown (the space frame 41 is shown in Figure 8 and is described below).

The branch portion 37 comprises an entrance 32 into the main lumen 16 within the intermediate portion 200 of tubular body 15. The branch portion 37 may have an exit 34 through the tubular wall 17 of the tubular body 15. The exit 34 through the tubular wall of the main lumen 16 may open into the recess or opening 250. The lumen 36 may run between the entrance 32 and the exit 34.

Referring now to Figure 7, which is an isometric view looking down into the endograft 10 from its proximal end 12, the branch portion 37 can be seen within the main lumen 16. The proximal end of the branch portion 37 may have a larger diameter than the distal end of the branch portion 37.

The deployment of one embodiment of the endograft 10 can occur in any vessel, duct, or lumen. The endograft 10 may be deployed in a patient using any known technique or method. In one example, the deployment device is the device disclosed in U.S. Pat. No. 7537606 titled *Branch stent graft deployment and method.*

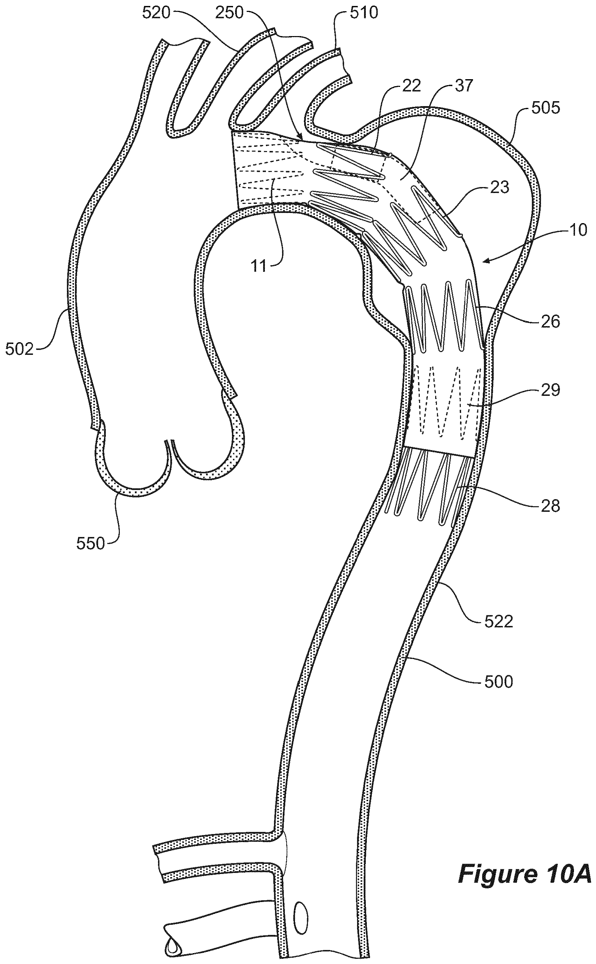

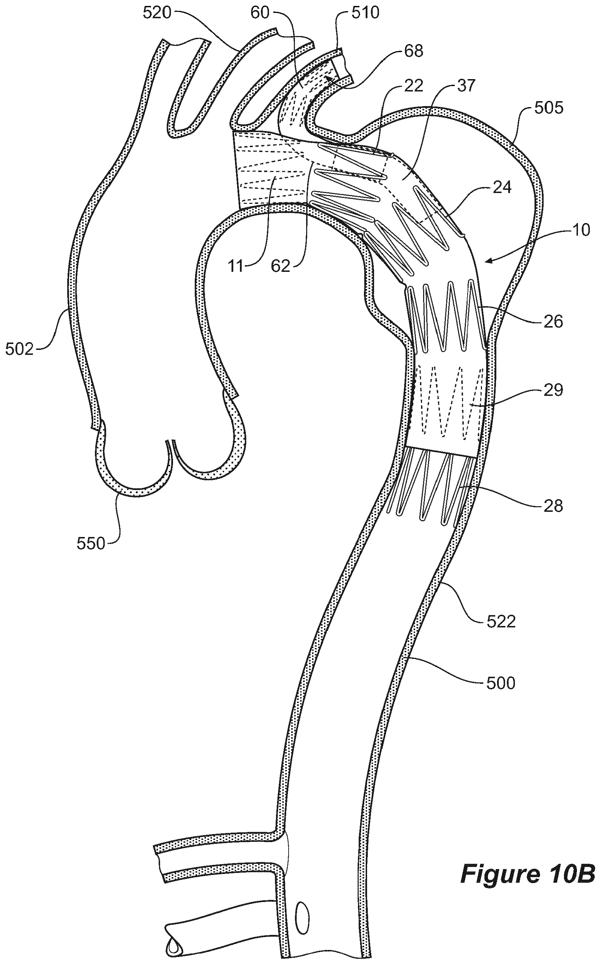

In one example, endograft 10 can be deployed into the thoracic arch of a patient. Figure 10A is a diagrammatic view of the endograft of Figures 5A, 6 and 7 within the anatomy of a patient. Figure 10B is a similar view to that of Figure 10A but also shows a side branch endograft.

The thoracic arch region of a patient generally comprises an ascending aorta 502 extending from an aortic valve 550 of the heart of the patient, then over the thoracic arch to the descending aorta 522. From the thoracic arch, three main arteries extend. These are the innominate artery, the left carotid artery 520 and a subclavian artery 510. This embodiment of the disclosure will generally be discussed with reference to deployment of an endograft 10 in the form of a stent graft 10 with a side branch into the aorta 500 and left subclavian artery 510 but the disclosure is not so restricted.

The endograft 10 may be necessary in the aortic arch region when an aneurysm in the aorta 500 extends up the aorta 500 to such an extent that there is insufficient patent aortic wall to provide good sealing for a stent graft 10 distally of the left subclavian artery 510 (for instance, see aneurysmal portion 505 in Figure 10A). It is desirable in such circumstances to extend the stent graft 10 to seal onto good artery wall at least between the left carotid artery 520 and the left subclavian artery 510.

The proximal end sealing region 11 is shown in Figures 10A and 10B sealing onto good artery wall at least between the left carotid artery 520 and the left subclavian artery 510. The distal end sealing region 29 is also shown in Figures 10A and 10B sealing onto good artery wall along the descending aorta 522 below the aneurysmal portion 505.

Figures 10A shows a detailed view of the deployed stent graft 10, as shown in Figure 5A, into a thoracic arch of a patient. A separate balloon expandable, or self expanding, side arm or side branch endograft 60 such as stent graft 60 can then be deployed brachially through the subclavian artery 510 so that its upstream or proximal end enters the branch lumen 36 and then it can be allowed to expand, or be balloon expanded, so that the proximal end is expanded within the branch portion 37 to seal the side branch stent graft 60 into the branch lumen 36 of the main stent graft 10, as is shown in Figure 10B. In other words, the recess or opening 250 may be adapted to accommodate a side arm, the side arm having an upstream end and a downstream end, the upstream end positionable within the branch lumen and the downstream end within a branch vessel. Again, this can be achieved as is described in the afore-mentioned U.S. Pat. No. 7537606 titled *Branch stent graft deployment and method.*

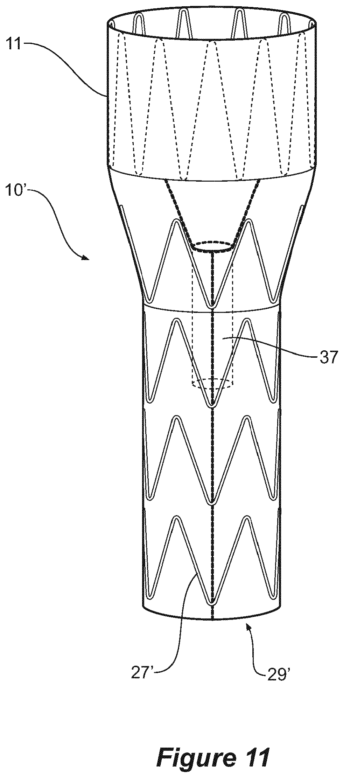

Figure 11 is an isometric view of another endograft 10' according to the disclosure. With this embodiment, the distal most stent 27' is an external stent. That is, the stent 27' is secured on the outside of the tubular body 15. This endograft 10' can be used as part of an interconnected stent graft assembly where a more distal stent graft seals to the inside of the distal end of the endograft 10'. Such an arrangement may be suitable for a patient who has an aneurysm that extends further down the descending aorta that the aneurysm 505 shown in Figures 10A and 10B.

The above described method of producing the endograft 10 utilises excess graft material 20 to produce the branch portion 37. This eliminates the need to separately fabricate and attach the branch portion 37, reducing the complexity of the final endograft 10 or endograft 10', as illustrated in Figures 5A and 11 respectively.

The disclosure is not limited to this particular application but is discussed in relation to this particular application as an example.

Embodiments of the disclosure will be used by vascular surgeons to treat aneurysms and to repair regions of the aorta, including, but not limited to, the aortic arch. Embodiments of the disclosure will be used in other parts of the vasculature system.

Throughout the specification and the claims that follow, unless the context requires otherwise, the words "comprise" and "include" and variations such as "comprising" and "including" will be understood to imply the inclusion of a stated integer or group of integers, but not the exclusion of any other integer or group of integers.

The reference to any prior art in this specification is not, and should not be taken as, an acknowledgement of any form of suggestion that such prior art forms part of the common general knowledge.

It will be appreciated by those skilled in the art that the disclosure is not restricted in its use to the particular application described. Neither is the present disclosure restricted in its preferred embodiment with regard to the particular elements and/or features described or depicted herein.

## Claims

1. An endograft comprising a proximal end and a distal end, the endograft comprising a tube of biocompatible material, the tube comprising:
a main lumen (16) extending longitudinally from a proximal end to a distal end, the main lumen (16) including a proximal portion, an intermediate portion and a distal portion;
a branch lumen (36) within the intermediate portion of the main lumen (16); and
a seam, the seam extending longitudinally beside both the branch lumen (36) and the distal portion of the main lumen (16),
wherein the tube comprises a lateral cross-section defining a tubular wall, wherein a portion of the tubular wall is pinched together by the seam to form an internal branch portion (37).

2. The endograft as claimed in claim 1 wherein the branch portion (37) comprises an entrance within the intermediate portion of the main lumen (16) and an exit through the tubular wall of the main lumen.

3. The endograft as claimed in claim 2 comprising a recess, wherein the exit through the tubular wall of the main lumen (16) opens into the recess.

4. The endograft as claimed in claim 3 wherein the tube is supported by a plurality of zig zag stents, each of the stents comprising a plurality of struts and bends, the bends being between adjacent struts,
wherein the recess is at least partly disposed within a V-shaped region formed between two adjacent struts and a bend of one of the plurality of zig zag stents.

5. The endograft as claimed in claim 4 wherein the tubular wall of the main lumen (16) comprises at least one seam adjacent the V-shaped region formed between two adjacent struts and a bend of one of the plurality of zig zag stents.

6. The endograft as claimed in any of claims 3 to 5 wherein the recess is adapted to accommodate a side arm, the side arm having an upstream end configured to be positioned and a downstream end, the upstream end positionable within the branch lumen (36) and the downstream end configured to be positioned within a branch vessel.

7. The endograft as claimed in any preceding claim wherein the branch portion (37) comprises an entrance reinforcing ring.

8. The endograft as claimed in any preceding claim wherein the branch portion (37) comprises an exit reinforcing ring.

9. The endograft as claimed in any preceding claim wherein the branch portion (37) comprises a reinforcement in the form of a space frame, the space frame formed by entrance and exit reinforcing rings spaced apart axially by at least one strut.

10. The endograft as claimed in claim 9 wherein the space frame comprises an assembly of two individual ring and strut components, each ring and strut component comprising a circular ring portion defining a plane of the circular ring portion and a strut extending at right angles to the plane of the circular ring portion from a periphery of the circular ring portion,
whereby one of the two circular rings forms the entrance reinforcing ring and the other of the two circular rings forms the exit reinforcing ring.

11. The endograft as claimed in claim 9 or 10 wherein the space frame comprises super-elastic wire.

12. The endograft as claimed in claim 11 wherein the super-elastic wire of the space frame comprises nitinol.

13. A method of producing a endograft comprising the steps of:
providing a tube of biocompatible material, the tube having a tubular wall;
flattening the tube so that the tubular wall forms two substantially planar wall portions;
partitioning the flattened tube by creating a longitudinal seam between the two wall portions;
cutting a piece out of the tube from a position adjacent to the longitudinal seam so as to create a branch mouth into the tube;
attaching a super-elastic ring to the branch mouth; and
everting the tube.

14. The method of claim 13, wherein the method comprises the steps of:
attaching a plurality of zig zag stents to the tubular wall; and/or
wherein the partitioning step comprises the step of:
sewing along the seam.

15. The method of claim 13 or 14, wherein the cutting step creates excess material terminating in edges, and wherein the method includes the step of sealing the excess material.

## Patentansprüche

1. Endograft, umfassend ein proximales Ende und ein distales Ende, wobei das Endograft eine Röhre aus biokompatiblem Material umfasst, wobei die Röhre umfasst:
ein Hauptlumen (16), das sich in Längsrichtung von einem proximalen Ende zu einem distalen Ende erstreckt, wobei das Hauptlumen (16) einen proximalen Abschnitt, einen Zwischenabschnitt und einen distalen Abschnitt einschließt;
ein Zweiglumen (36) innerhalb des Zwischenabschnitts des Hauptlumens (16); und
eine Naht, wobei die Naht sich in Längsrichtung neben sowohl dem Zweiglumen (36) als auch dem distalen Abschnitt des Hauptlumens (16) erstreckt,
wobei die Röhre einen seitlichen Querschnitt umfasst, der eine Röhrenwand definiert, wobei ein Abschnitt der Röhrenwand durch die Naht zusammengekniffen wird, um einen inneren Zweigabschnitt (37) zu bilden.

2. Endograft nach Anspruch 1, wobei der Zweigabschnitt (37) einen Eingang innerhalb des Zwischenabschnitts des Hauptlumens (16) und einen Ausgang durch die Röhrenwand des Hauptlumens umfasst.

3. Endograft nach Anspruch 2, der eine Aussparung umfasst, wobei der Ausgang sich durch die Röhrenwand des Hauptlumens (16) in die Aussparung öffnet.

4. Endograft nach Anspruch 3, wobei die Röhre durch eine Vielzahl von Zickzack-Stents gestützt wird, wobei jeder der Stents eine Vielzahl von Streben und Biegungen umfasst, wobei die Biegungen zwischen benachbarten Streben liegen,
wobei die Aussparung mindestens teilweise innerhalb einer V-förmigen Region angeordnet ist, die zwischen zwei benachbarten Streben und einer Biegung von einer der Vielzahl von Zickzack-Stents gebildet ist.

5. Endograft nach Anspruch 4, wobei die Röhrenwand des Hauptlumens (16) mindestens eine Naht benachbart zu der V-förmigen Region umfasst, die zwischen zwei benachbarten Streben und einer Biegung von einer von der Vielzahl der Zickzack-Stents gebildet ist.

6. Endograft nach einem der Ansprüche 3 bis 5, wobei die Aussparung eingerichtet ist, um einen Nebenarm unterzubringen, wobei der Nebenarm ein stromaufwärtiges Ende, das ausgestaltet ist, um positioniert zu werden, und ein stromabwärtiges Ende aufweist, wobei das stromaufwärtige Ende innerhalb des Zweiglumens (36) positionierbar ist und das stromabwärtige Ende ausgestaltet ist, um innerhalb eines Zweiggefäßes positioniert zu werden.

7. Endograft nach einem der vorhergehenden Ansprüche, wobei der Zweigabschnitt (37) einen Eingangsverstärkungsring umfasst.

8. Endograft nach einem der vorhergehenden Ansprüche, wobei der Zweigabschnitt (37) einen Ausgangsverstärkungsring umfasst.

9. Endograft nach einem der vorhergehenden Ansprüche, wobei der Zweigabschnitt (37) eine Verstärkung in Form eines Raumrahmens umfasst, wobei der Raumrahmen durch Eingangs- und Ausgangsverstärkungsringe gebildet ist, die axial um mindestens eine Strebe beabstandet sind.

10. Endograft nach Anspruch 9, wobei der Raumrahmen eine Anordnung von zwei individuellen Ring- und Strebenkomponenten umfasst, jede Ring- und Strebenkomponente einen kreisförmigen Ringabschnitt, der eine Ebene des kreisförmigen Ringabschnitts definiert, und eine Strebe umfasst, die sich von einer Peripherie des kreisförmigen Ringabschnitts in rechten Winkeln zu der Ebene des kreisförmigen Ringabschnitts erstreckt,
wobei einer von den zwei kreisförmigen Ringen den Eingangsverstärkungsring und der andere von den zwei kreisförmigen Ringe den Ausgangsverstärkungsring bildet.

11. Endograft nach Anspruch 9 oder 10, wobei der Raumrahmen superelastischen Draht umfasst.

12. Endograft nach Anspruch 11, wobei der superelastische Draht des Raumrahmens Nitinol umfasst.

13. Verfahren zur Produktion eines Endografts, umfassend die Schritte:
Bereitstellen einer Röhre aus biokompatiblem Material, wobei die Röhre eine Röhrenwand aufweist;
Abflachen der Röhre, so dass die Röhrenwand zwei im Wesentlichen planare Wandabschnitte bildet;
Partitionieren der abgeflachten Röhre, indem eine längsgerichtete Naht zwischen den beiden Wandabschnitten erzeugt wird;
Herausschneiden eines Stücks aus der Röhre von einer Position benachbart zu der längsgerichteten Naht, um so eine Zweigmündung in der Röhre zu erzeugen;
Anbringen eines superelastischen Rings an der Zweigmündung; und
Umstülpen der Röhre.

14. Verfahren nach Anspruch 13, wobei das Verfahren die Schritte umfasst:
Anbringen einer Vielzahl von Zickzack-Stents an der Röhrenwand; und/oder
wobei der Partitionierungsschritt den Schritt umfasst:
Nähen entlang der Naht.

15. Verfahren nach Anspruch 13 oder 14, wobei der Schneidschritt Überschussmaterial erzeugt, das in Rändern endet, und wobei das Verfahren den Schritt des Versiegelns des Überschussmaterials einschließt.

## Revendications

1. Endogreffe comprenant une extrémité proximale et une extrémité distale, l'endogreffe comprenant un tube de matériau biocompatible, le tube comprenant :
une lumière principale (16) s'étendant longitudinalement d'une extrémité proximale à une extrémité distale, la lumière principale (16) comprenant une partie proximale, une partie intermédiaire et une partie distale ;
une lumière secondaire (36) à l'intérieur de la partie intermédiaire de la lumière principale (16) ; et
une couture, la couture s'étendant longitudinalement à côté à la fois de la lumière secondaire (36) et de la partie distale de la lumière principale (16),
dans laquelle le tube comprend une section transversale latérale définissant une paroi tubulaire, dans laquelle une partie de la paroi tubulaire est pincée par la couture pour former une partie secondaire interne (37).

2. Endogreffe selon la revendication 1 dans laquelle la partie secondaire (37) comprend une entrée à l'intérieur de la partie intermédiaire de la lumière principale (16) et une sortie à travers la paroi tubulaire de la lumière principale.

3. Endogreffe selon la revendication 2 comprenant un renfoncement, dans laquelle la sortie à travers la paroi tubulaire de la lumière principale (16) débouche dans le renfoncement.

4. Endogreffe selon la revendication 3 dans laquelle le tube est supporté par une pluralité d'endoprothèses en zigzag, chacune des endoprothèses comprenant une pluralité d'entretoises et de coudes, les coudes se trouvant entre des entretoises adjacentes,
dans laquelle le renfoncement est au moins partiellement disposé à l'intérieur d'une région en forme de V formée entre deux entretoises adjacentes et un coude d'une endoprothèse de la pluralité d'endoprothèses en zigzag.

5. Endogreffe selon la revendication 4 dans laquelle la paroi tubulaire de la lumière principale (16) comprend au moins une couture adjacente à la région en forme de V formée entre deux entretoises adjacentes et un coude d'une endoprothèse de la pluralité d'endoprothèses en zigzag.

6. Endogreffe selon l'une quelconque des revendications 3 à 5 dans laquelle le renfoncement est conçu pour accueillir un bras latéral, le bras latéral ayant une extrémité amont configurée pour être positionnée et une extrémité aval, l'extrémité amont pouvant être positionnée à l'intérieur de la lumière secondaire (36) et l'extrémité aval étant configurée pour être positionnée à l'intérieur d'un vaisseau secondaire.

7. Endogreffe selon une quelconque revendication précédente dans laquelle la partie secondaire (37) comprend un anneau de renforcement d'entrée.

8. Endogreffe selon une quelconque revendication précédente dans laquelle la partie secondaire (37) comprend un anneau de renforcement de sortie.

9. Endogreffe selon une quelconque revendication précédente dans laquelle la partie secondaire (37) comprend un renfort sous la forme d'un cadre spatial, le cadre spatial étant formé par des anneaux de renfort d'entrée et de sortie espacés axialement par au moins une entretoise.

10. Endogreffe selon la revendication 9 dans laquelle le cadre spatial comprend un assemblage de deux composants individuels d'anneau et d'entretoise, chaque composant d'anneau et d'entretoise comprenant une partie d'anneau circulaire définissant un plan de la partie d'anneau circulaire et une entretoise s'étendant à angle droit par rapport au plan de la partie d'anneau circulaire à partir d'une périphérie de la partie d'anneau circulaire,
moyennant quoi l'un des deux anneaux circulaires forme l'anneau de renforcement d'entrée et l'autre des deux anneaux circulaires forme l'anneau de renforcement de sortie.

11. Endogreffe selon la revendication 9 ou 10 dans laquelle le cadre spatial comprend un fil superélastique.

12. Endogreffe selon la revendication 11 dans laquelle le fil superélastique du cadre spatial comprend du nitinol.

13. Procédé de fabrication d'une endogreffe comprenant les étapes de :
fourniture d'un tube en matériau biocompatible, le tube ayant une paroi tubulaire ;
aplatissement du tube de sorte que la paroi tubulaire forme deux parties de paroi sensiblement planes ;
cloisonnement du tube aplati en créant une couture longitudinale entre les deux parties de paroi ;
découpage d'un morceau du tube à partir d'une position adjacente à la couture longitudinale de sorte à créer une embouchure secondaire dans le tube ;
fixation d'un anneau superélastique à l'embouchure secondaire ; et
retournement du tube.

14. Procédé selon la revendication 13, le procédé comprenant les étapes de :
fixation d'une pluralité d'endoprothèses en zigzag à la paroi tubulaire ; et/ou
dans lequel l'étape de cloisonnement comprend l'étape de :
couture le long de la couture.

15. Procédé selon la revendication 13 ou 14, dans lequel l'étape de découpage crée du matériau excédentaire se terminant par des bords, et dans lequel le procédé comprend l'étape de scellement du matériau excédentaire.
